# EUROPEAN PATENT APPLICATION

(11) **EP 2 223 662 A1**
(43) Date of publication of application: **01.09.2010**
(21) Application number: 08852740.3
(22) Date of filing: 20.11.2008
(51) Int. Cl.: A61B 17/34

(54) **ELECTRONIC SYRINGE WITH SAFETY SYSTEM FOR SPINAL INJECTIONS**

(30) Priority: 20.11.2007 US 989333 P; 20.11.2008 US 275162
(71) Applicant: Innovamédica S.A.P.I. DE C.V., C.P. 14050 México, D.F. (MX)
(72) Inventor: SACRISTÁN ROCK, Emilio, C.P. 14400 México, D.F. (MX); SILVA PAREDES, Alejandra, C.P. 14210 México, D.F. (MX); RODRIGUEZ BERMÚDEZ, Miroslava Carolina, C.P. 06070 México, D.F. (MX); BECERRIL ALQUICIRA, Adriana, C.P. 16500 México, D.F. (MX); ORANDAY MUÑOZ, Lydia, C.P. 09470 México, D.F. (MX); SORIA AGUILAR, Julio César, C.P. 15850 México, D.F. (MX)
(74) Representative: McLean, Robert Andreas
(86) International application number: PCT/MX2008/000158
(87) International publication number: WO 2009/066972

(57) **Abstract**

A device for locating an epidural space. The device includes a connector for forming a hermetically sealed connection with a needle. An air vessel is formed by an air tank, a diaphragm, and a space in the needle when the needle is attached to the connector. The device includes a pressure sensor configured to detect the air pressure in the air vessel. An actuator is coupled to a switch and positioned adjacent to the diaphragm to compress the diaphragm when the switch is actuated by a user causing a reduction in volume in the air vessel. A circuit coupled to the pressure sensor and to an alarm detects a signal indicative of the air pressure sensed by the pressure sensor. The circuit compares the signal to a threshold and outputs a first state to the alarm when the pressure is above the threshold, and outputs a second state to the alarm when the pressure falls below the threshold.

## Description

### RELATED APPLICATIONS

This application claims priority of U.S. Provisional Patent Application Serial Number 60/989,333, titled ELECTRONIC SYRINGE WITH SAFETY SYSTEM FOR SPINAL INJECTION, and filed on November 20, 2007, which is incorporated by reference in this application in its entirety.

### BACKGROUND

### 1. Field of the Invention.

This invention relates to epidural needles, and more particularly to epidural needles used for spinal injections.

### 2. Related Art.

Advances in perioperative anesthesia and analgesia have improved pain relief and satisfaction in surgical patients. Recent studies suggest that advances in anesthesia and postoperative analgesia can affect postoperative outcome. Epidural anesthesia and analgesia have the potential to reduce or eliminate the perioperative stress responses to surgery and thereby decrease surgical complications and improve outcomes while reducing hospital costs.

Epidural anesthesia has been used by physicians for about a century. In 1901, Radiologist Jean-Anthanase Sicard reported treating patients suffering from severe intractable sciatic pain or lumbago by injecting diluted solutions of cocaine through the sacral hiatus. Physicians began to use a lumbar approach to epidural anesthesia several decades later, eventually in abdominal surgery with single - shot lumbar epidural anesthesia.

The epidural space is the part of the vertebral canal not occupied by the dura mater and its contents. It is a very narrow space, often referred to as a 'potential space,' that lies between the dura and the periosteum lining inside the vertebral canal. The dura and ligamentum flavum are closely adjacent the potential space. The epidural space extends from the foramen magnum to the sacral hiatus. The anterior and posterior nerve roots in their dural covering pass across this potential space to unite in the intervertebral foramen to form segmental nerves. The epidural space contains venous plexuses and fatty tissue which is continuous with the fat in the paravertebral space.

The use of epidural anesthesia has expanded widely over the years. Epidural anesthesia can be used as the sole anesthetic for procedures involving the lower limbs, pelvis, perineum and lower abdomen. It is possible to perform upper abdominal and thoracic procedures under epidural anesthesia alone. Some of the specific procedures in which epidural anesthesia may be used include: hip and knee surgery, vascular reconstruction or amputation of the lower limbs, obstetrics, low concentration local anesthetics, opioids, or combination of both, and thoracic trauma with rib or sternum fractures.

The target population for use of epidural anesthesia includes pregnant women undergoing a cesarean section, or women at term who are planning a vaginal delivery but wish to have an epidural anesthetic available for labor pain management.

An added advantage of using epidural anesthesia is the ability to maintain continuous anesthesia after placement of an epidural catheter, making it suitable for procedures of long duration. This feature also enables the use of this technique into the postoperative period for analgesia, using lower concentrations of local anesthetic drugs or in combination with different agents.

When an epidural block is performed, the epidural space is entered by the tip of the needle after it passes through the ligamentum flavum. The epidural needle follows an anatomical direction from the skin to into the body, through the subcutaneous tissue, ligamentum interspinous, flavum and arriving then to the epidural space. The user identifies the space and enters it carefully as the bevel of the needle exits the ligamentum flavum to avoid penetrating the dura if the needle is advanced to far. The epidural space ends in the sacrococcygeal ligamentum. The largest distance is found in the medial line and is about 5 mm. When the epidural space is found, the anesthesia is injected into the space. The epidural anesthetic molecules surround nerve roots and penetrate deeply into the spinal cord tissue, preventing sodium channel closing, thus blocking pain message transmission from the site of injection and below.

One way to identify the epidural space during an epidural anesthesia procedure involves identifying a negative pressure to the advancing needle. The pressure in the epidural space has been measured to be a high negative epidural pressure of up to - 60 mm Hg at the moment of epidural puncture. However, the pressure typically becomes positive and stabilized at +3.7 ± 3.2 mmHg. This suggests that negative epidural pressures at the moment of epidural puncture may be due to tenting of the dural membrane. Air and saline are typically put in the syringes attached to the epidural needles to determine the loss of resistance during the insertion of an epidural. On the other hand, there may not be any negative pressure at all in some cases, or the negative pressure may not be significant.

The ability to correctly identify the epidural space may enhance the benefits of epidural anesthesia by minimizing complications. Complications that may arise during an epidural procedure include: bony resistance, inability to treat the catheter, fluid through the needle, fluid through the catheter, and pain on insertion of the catheter or blood in the catheter. The most significant hazard of epidural blockade is unrecognized, unintentional intravascular injection. These complications are more probable with epidural anesthesia than with other regional techniques because of the number of plexuses in the epidural space, and possibly because of the relatively low pressure existing in these veins.

Another potential complication in epidural anesthesia is air embolism. The air volume injected into the epidural veins may produce slight clinical manifestations of around 0.07 mL per weight kilogram. Although the usual volume of injected air may not be dangerous, the persistence of the permeable foramen oval may result in an unexpected symptomatology. This may be indicated by the traumatic and accidental punction directly to the venous epidural plexus during the maneuver of detection of the epidural space. Air is as likely as the substance injected in the epidural space to pass to the circulatory system within 15 seconds due to the pressure difference. An air embolism of micro bubbles may also flow towards the general circulation.

Accidental dural puncture is typically recognized by the immediate loss of cerebral-spinal fluid (CSF) through the epidural needle. Accidental dural puncture leads to a high incidence of post dural puncture headache, which may be quite severe. Accidental dural puncture may occur even when the procedure is performed by highly skilled physicians. It is more common when performed by more inexperienced hands.

The afore-mentioned complications occur less frequently when the procedures are performed with more skilled doctors. However, doctors with sufficient expertise may not be readily available at any given time. Moreover, it would be desirable to perform epidural blocks and anesthesia without relying on the doctor's expertise.

In view of the above-described complications and risks, the localization of the epidural space is critical to the success of the epidural anesthesia. Devices and procedures have been designed to improve the procedure. Currently, there are two basic techniques for identifying when the needle enters the epidural space: one based on the loss of resistance detected when the needle enters the space, and the other that detects the change of pressure inside the space.

The loss of resistance technique is the most common method for locating a needle in the epidural space. When a Tuohy needle is introduced into the epidural space, the anesthesiologist is able to feel the loss of resistance to injection through the needle. This may be due to the presence of a negative pressure in the epidural space. As noted above, the negative pressure may not always be present. In such cases, loss of resistance is not due to negative pressure, and use of the technique may feel different for the user.

Over the years, other instruments or techniques based on the basic principles of the two theories for identifying the epidural spaces (the loss of resistance and the negative pressure) have been developed in order to complement or replace the original techniques. Examples include:
1. The Macintosh ball, based on the principle of loss of resistance, consists of a small elastic rubber ball, which is permanently inflated with air while the needle is in the ligamentum flavum. The Macintosh ball is deflated when it enters the epidural space. One problem that has been observed with this device is a higher incidence of incomplete anesthesia due to the quantity of air injected.
2. An infusion dripping connected to the Tuohy needle. This technique is based on the difference of pressure between the atmospheric pressure and the pressure inside the epidural space. Once the Tuohy needle is introduced in the interspinal ligament, the stylet tip is extracted and connected to an infusion serum system in the distal end of the needle. The velocity of the dripping will indicate the position of the needle top. A sudden change in the dripping velocity, such as a free flow, indicates that the needle has penetrated the epidural space. This method is not precise and may be more effective at the thoracic level due to the negative pressure. With this technique, it is not possible to find the difference between the epidural space and the subarachnoid space because the infusion will continue on free dropping, even if the needle has crossed the dura mater.
3. The Episensor^{™}, which is an electronic method for detecting the negative pressure in the epidural space. This device has met with results that may be no better than more traditional techniques.
4. A device that includes a syringe with two chambers (known as the Epident®). The device has a distal chamber, which contains 3 mL of saline solution and a proximal chamber containing air. The device has the compressibility sensation that a user desires, avoiding the inconvenience of the loss of resistance that occurs with the air technique. Similar syringes have been designed with a diaphragm, which is used to separate both fluids. The system is similar to the air bubble method, but is more complex and expensive.
5. A glass syringe with a plunger, which slides very easily, has been used to identify the epidural spaces. Newer commercially available disposable epidural packs contain a plastic syringe with a plunger that has very low resistance. Normal syringes should not be used because their greater resistance may make identification of the epidural space more difficult.

These solutions for locating the epidural space have been designed to offer comfort to the operator while reducing complications. Nevertheless, they have met with limited success.

There remains a need for an automatic electronic device that provides an objective approach to the identification of the epidural space that is safe, easy to use and has a sensitive and specific end point when producing a positive result.

### SUMMARY

In view of the above, a device is provided for locating the epidural space. The device includes a connector for forming a hermetically sealed connection with a needle. An air vessel is formed by an air tank, a diaphragm, and a space in the needle when the needle is attached to the connector. The device includes a pressure sensor configured to detect the air pressure in the air vessel. An actuator is coupled to a switch and positioned adjacent to the diaphragm to compress the diaphragm when the switch is actuated by a user causing a reduction in volume in the air vessel. A circuit coupled to the pressure sensor and to an alarm detects a signal indicative of the air pressure sensed by the pressure sensor. The circuit compares the signal to a threshold and outputs a first state to the alarm when the pressure is above the threshold, and outputs a second state to the alarm when the pressure falls below the threshold.

In another aspect of the invention, a method is provided for locating an epidural space. The method includes inserting a needle in a patient until sensing the ligamentum flavum. A closed air vessel is formed with an air tank and the space in the needle. The volume in the air vessel is reduced, and the air pressure in the air vessel is measured. The air pressure measurement is compared with a threshold while advancing the needle into the patient. When the air pressure falls below the threshold, advancement of the needle is stopped by the user.

Other systems, methods and features of the invention will be or will become apparent to one with skill in the art upon examination of the following figures and detailed description. It is intended that all such additional systems, methods, features and advantages be included within this description, be within the scope of the invention, and be protected by the accompanying claims.

### BRIEF DESCRIPTION OF THE DRAWINGS

The example implementations of the invention described below may be better understood by referring to the following figures. The components in the figures are not necessarily to scale, emphasis instead being placed upon illustrating the principles of the invention. In the figures, like reference numerals designate corresponding parts throughout the different views.

FIG. 1 is a perspective view of an electronic syringe.

FIG. 2 is an exploded view of the electronic syringe of FIG. 1.

FIG. 3 is a side cross-sectional view of the electronic syringe of FIG. 1.

FIG. 4 is a perspective view of the electronic syringe of FIG. 1 with part of the housing removed.

FIG. 5 is a schematic diagram of the electronic circuit used in the electronic syringe in FIG. 1.

FIG. 6 is a flowchart illustrating operation of the electronic syringe.

### DETAILED DESCRIPTION

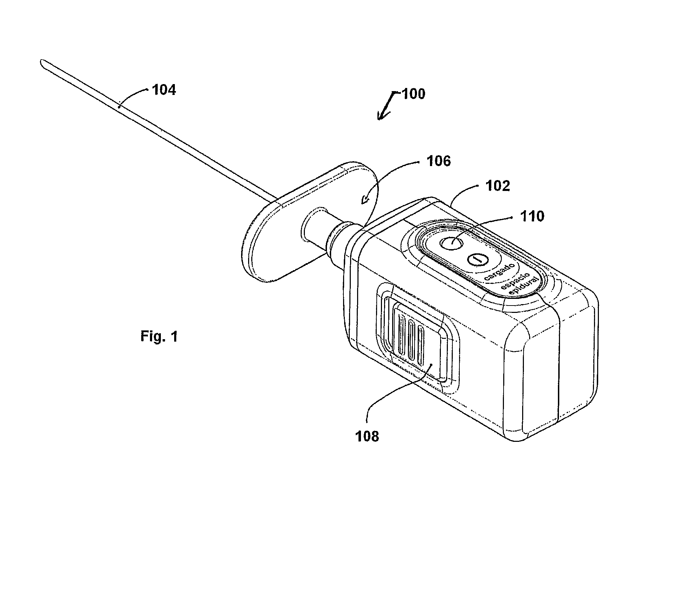

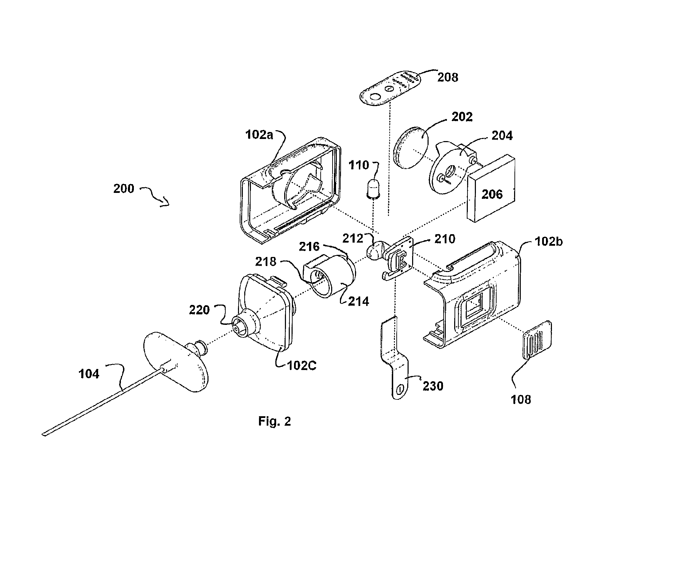

FIG. 1 is a perspective view of an example of an electronic syringe 100. The electronic syringe 100 in FIG. 1 includes a housing 102, which holds the internal components (described in more detail below with reference to FIGs. 2-5), and a needle 104. The electronic syringe 100 also includes a connector 106 for connecting the needle 104, which may include a stylet tip, a switch 108 for activating the electronic syringe 100, and an alarm 110 to indicate when the epidural space has been entered.

The electronic syringe 100 is used by a doctor, or anesthesiologist or any other trained medical personnel, to locate the epidural space during an anesthesia procedure. The electronic syringe 100 facilitates the punction into the epidural space without advancing the needle too deep. A built-in safety mechanism in the form of a pneumatic sensor generates an alarm when the user is at risk of advancing too far. The user operates the electronic syringe 100 as an anatomical pressure localizer to precisely locate the epidural space advantageously minimizing complications such as a dura punction, which may lead to a release of spinal fluid during the anesthesia procedure.

The electronic syringe 100 in FIG. 1 may be implemented in a size that would permit use as a hand-held device used by a single person. In one example, the electronic syringe 100 is approximately 36.5 W x 35.4 T x 59 L mm, however, any the electronic syringe 100 may be any suitable size. The examples of the electronic syringe 100 described here are battery-powered; however, any form of electrical power may be used. A feature may be included to ensure that the battery is only activated for an intended first use by avoiding accidental activation. For example, a small piece of paper may be inserted between the battery and its contacts such that it extends out of the housing 102 through slit to allow the user to withdraw the paper to enable the first use. Once the paper is cleared, the user may initiate operation by sliding the switch 108 to activate the electronic syringe 100. In the illustrated example, the electronic syringe 100 includes two lithium 3V batteries in series that are capable of powering the device for around one hour.

The alarm 110 in the example shown in FIG. 1 is a visual alarm 110 implemented as an LED, or other suitable light emitting device. The alarm 110 may also be implemented as an audible alarm, or any other device intended to attract the user's attention and indicate the occurrence of an event. The alarm 110 indicates when the user has reached the epidural space. In one example electronic syringe 100, the alarm 110 is implemented as an LED that changes to a red color to indicate that the needle may be advanced, and to a green color to indicate that the epidural space has been reached.

The electronic syringe 100 includes the connector 106, which may be a Luer-type connector to provide a hermetically sealed connection to the needle 104. Although, any suitable connector 106 may be used. The examples of the electronic syringe 100 are described here as being compatible for use with a Tuohy needle 104; however, any suitable needle 104 may be used. The needle 104 used with the electronic syringe 100 may include a stylet tip; however, any suitable structure or technique may be used to prevent tissue from entering the needle 104 as it is inserted into the patient. In one example, a stylet tip may be integrated with the needle 104 and attached to the electronic syringe 100 as an assembly. In another example, no stylet tip is used with the needle 104 during the procedure.

The electronic syringe 100 may be used as follows. The user first introduces the Tuohy needle 104 into the patient. Initially, the needle 100 may be inserted with a stylet tip. Neither the needle 104 nor stylet are attached to the electronic syringe 100 when first introduced into the patient. The user inserts the needle and stylet 104 until reaching the ligamentum flavum. When the ligamentum flavum is reached, the stylet tip is retired and the electronic syringe 100 is connected to the Tuohy needle 104 at the Luer connector 106 creating a hermetically sealed connection. The user then slides the switch 108 to the active state. A pressure sensor inside the housing 102 detects a pressure inside an air tank continuously, and the pressure is compared by an electronic circuit with a threshold pressure. As long as the measured pressure is greater than the threshold level, the alarm 110 is set to indicate that the user may continue to advance the needle into the patient. When the epidural space is reached, a sudden decrease in pressure below the threshold is detected and the electronic circuit causes the alarm 110 to indicate that the epidural space is reached.

One advantage of the electronic syringe 100 in FIG. 1 is that the alarm 110 provides built-in fault detection. For example, when the electronic syringe 100 is connected to the needle at the Luer connector 106, if the alarm 110 is not set to indicate that the user may advance the needle after the switch 108 is slid into the active state, the user does not advance the needle. If the alarm 110 does not change, there could be a problem with the devices or the needle may not have been inserted properly.

In another example of the use of the electronic syringe 100, the needle 104, and an attached stylet tip, may be connected to the electronic syringe 100 prior to inserting into the patient. The user may then insert the needle attached to the electronic syringe 100 into the patient and slide the switch 108 to charge the electronic syringe 100. Assuming the alarm 110 indicates that the needle may be advanced, the user continues the insertion of the needle until the alarm 110 changes to indicate that the epidural space has been reached. In this example, the stylet tip is fixed to the needle 104 as an attachment to the electronic syringe 100. The stylet tip has a smaller caliber than the Tuohy needle 104 and may include an airway to allow air to escape through the needle 104 when the needle 104 is first applied to the patient. The airway may then become obstructed when the needle 104 is introduced further into the patient thereby closing the space in the needle 104 and tank of the electronic syringe 100. By adding the stylet tip to the needle 104 while attached to the electronic syringe 100, it is not necessary to first insert the stylet tip into the patient to detect the yellow ligament. The electronic syringe 100 may be used as one unit from the moment the needle 104 is inserted into the patient.

FIG. 2 is an exploded view of the electronic syringe of FIG. 1. The exploded view 200 in FIG. 2 shows the needle 104 removed from an exploded view of the housing 102 its contents. The housing 102 is shown in its component parts, a first side housing 102a, a second side housing 102b, and a housing cap 102c. Inside the housing 102 are components that include two batteries 202, a battery holder 204, a circuit board 206, an air tank 214, a diaphragm 216, a pressure sensor 218, an actuator 212, and an internal switch mechanism 210. The actuator 212 is mounted in the switch mechanism 210 to follow the motion of the switch 108. The actuator 212 is positioned to be in slight contact, or very close to, the diaphragm 216.

The air tank 214 contains air and is hermetically sealed to the housing cap 102c. When the needle 104 is inserted into the patient, a closed air space is formed in the air tank 214 and in the needle 104. The diaphragm 216 may be made of a compressible material. When the switch 108 is slid to activate the electronic syringe 100, the diaphragm 216 is compressed causing a reduction of volume in the closed space formed by the air tank 214 and needle 104. The compression of the diaphragm 216 charges the air tank 214 by increasing pressure that is higher than a threshold pressure. In one example, the threshold may be set to about 20 mm Hg within about ±2 mm Hg. The pressure sensor 218 continuously monitors the air pressure in the air tank 214. When the epidural space is found, it provides a slight escape for the air in the air tank 214 sufficient to decrease the air pressure in the air tank 214. The alarm 110 indicates when the epidural space has been found by changing color when the pressure drops in the air tank 214.

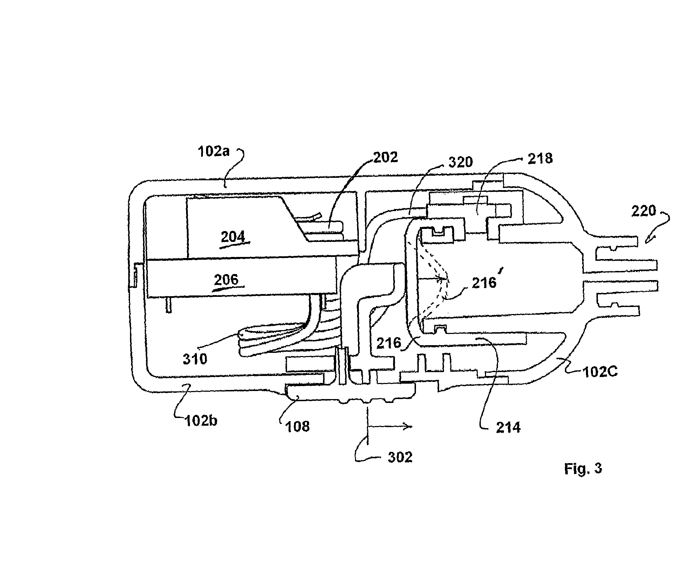

FIG. 3 is a side cross-sectional view of the electronic syringe of FIG. 1. The view in FIG. 3 is taken such that the switch 108 is on the bottom side of the housing 102b. As shown in FIG. 3, the battery 202 sits in the battery holder 204 and the battery holder 204 is mounted on top of the circuit board 206. A set of wires 310 extends from the circuit board 206 to make electrical connection with the pressure sensor 218 and the alarm 110 (shown in FIGs. 1 and 2). The view in FIG. 3 illustrates the motion of the switch 108 at 302 when the electronic syringe 100 is activated. The switch 108 causes the actuator 212 to press on the diaphragm 216 to a compressed state at 216'. The switch 108 is activated while the needle (and/or stylet tip) has been inserted partially into the patient creating a higher pressure from the decreased volume in the air tank 214. When the epidural space is reached, the air in the air tank 214 is released into the epidural space causing a decrease in pressure in the air tank 214. The pressure sensor 218 senses the drop in pressure and generates a signal to the alarm 110 to indicate that the epidural space has been reached.

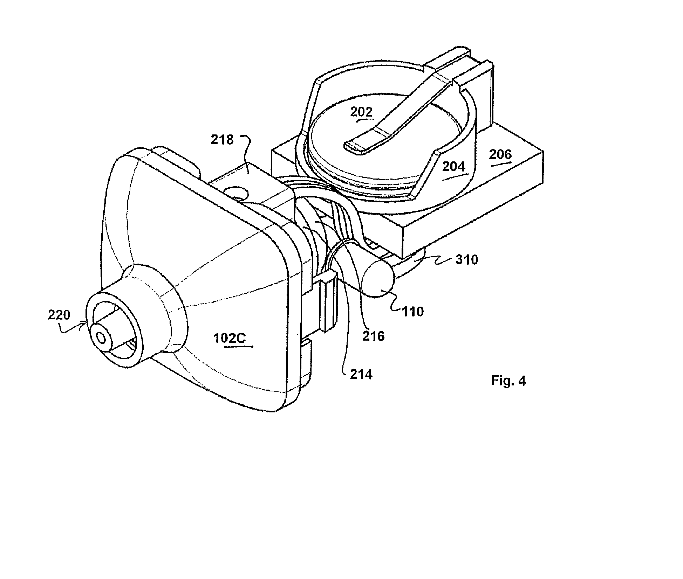

FIG. 4 is a perspective view of the electronic syringe of FIG. 1 with part of the housing removed. FIG. 4 shows the battery 202, the battery holder 204, the circuit board 206, and the wires 310 from the circuit board 206 to the alarm 110 and the pressure sensor 218. The pressure sensor 218 is shown positioned on the air tank 214. The diaphragm 216 is shown sealing the rear end of the air tank 214.

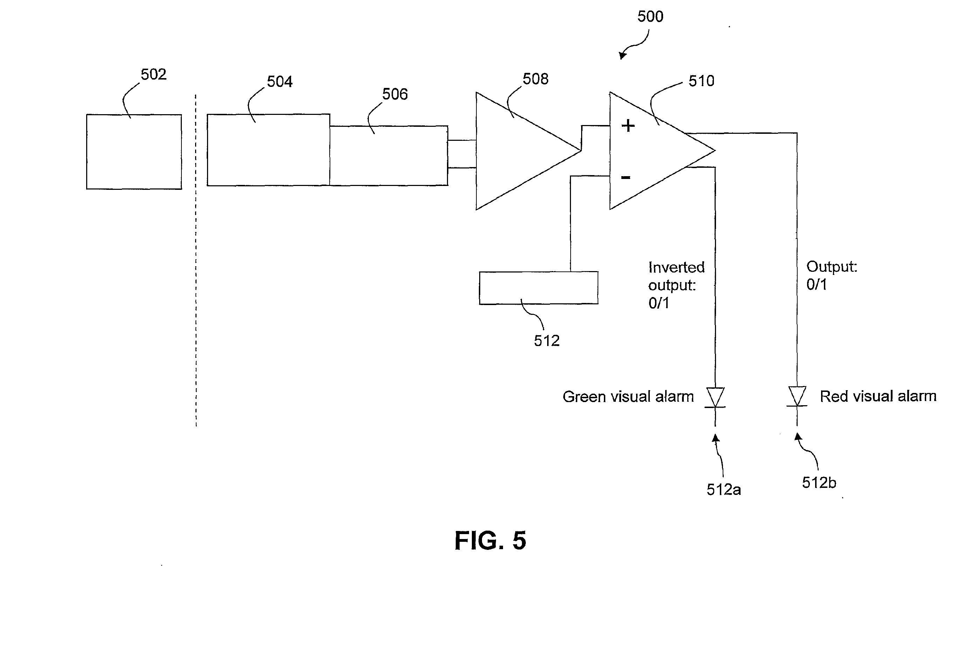

FIG. 5 is a schematic diagram of an electronic circuit 500 used in the electronic syringe in FIG. 1. The electronic circuit 500 includes a Tuohy needle 502, an air vessel 504, a pressure sensor 506, an amplifier 508, a comparator 510, a threshold indicator 512, and a comparator output 512. In operation, the Tuohy needle 502 is inserted into the patient. When the electronic syringe 100 is attached to the needle 502, an air vessel 504 is formed in the air tank 214 and the space of the needle 502. The air vessel 504 is a closed volume and when the electronic syringe 100 is charged by activating the switch 108, the pressure sensor 506 detects the increase in pressure over about 35 mm Hg. The pressure sensor 506 may have a range of about 0 to 300 mm Hg. The pressure sensor 506 outputs an electrical signal that is indicative of the pressure sensed to the amplifier 508. The amplifier 508 outputs an amplified signal to the comparator 510. The amplified signal is compared to the threshold 512, which is an electronic signal indicative of a threshold pressure below which is indicative of having reached the epidural space. As long as the comparator output 510 indicates that the pressure in the air vessel 504 is greater than the threshold 512, a red visual alarm 512a is output. When the comparator 510 output changes states indicating that the pressure in the air vessel 504 has dropped below the threshold, the comparator 510 output a signal that changes the visual alarm to a green color 512b. When the user detects the change to the green color 512b, the user stops advancing the needle avoiding puncturing the dural.

It is to be understood that the color changes in the visual alarm are red and green to illustrate operation of the device. Any color change may be used. In addition, an audible alarm may be added to the device, or used instead of a visual alarm.

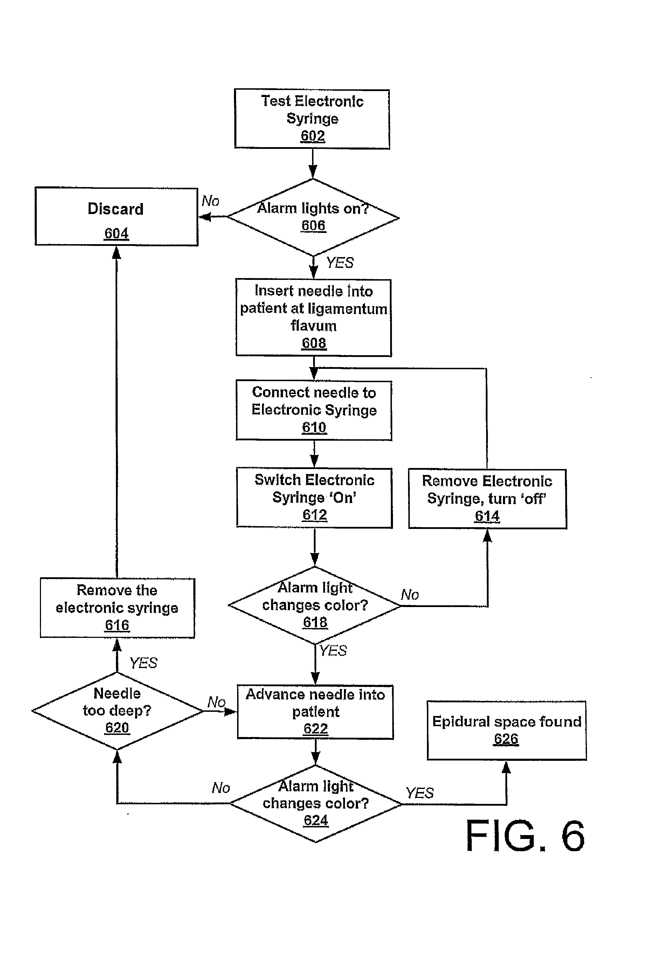

FIG. 6 is a flowchart illustrating operation of the electronic syringe 100 (in FIG. 1). The method may begin by a test of the electronic syringe 100 to determine if it works at step 602. Any suitable test may be performed. In one example, the user presses the switch 108 (FIG. 1) while blocking the air escape at the connector to prevent air escaping the air tank to activate the electronic syringe 100. The press of the switch 108 should turn on the alarm, which in this example is assumed to be a light, either green or red. The user may then unblock the air escape from the air tank, allowing the air to escape. As the air escapes the air tank the alarm should change color, for example, either red or green. The switch is returned to the inactive state once the test is complete.

Decision block 606 checks if the indicator light went on when the switch was pressed. If the indicator light did not go 'on,' the electronic syringe 100 is discarded at step 604. If the indicator light went 'on,' the needle is placed into the patient and pressed until reaching the ligamentum flavum at step 608. At step 610, the needle is connected to the electronic syringe 100. In one example, a Luer connector is used to make the connection. At step 612, the button on the electronic syringe 100 is pressed to the active state. Decision block 618 checks if the alarm shows a color change, for example to red, or from green to red. If the color did not change when the button was pressed, the electronic syringe 100 is removed from the needle at step 614, and the button is returned to the inactive state. The electronic syringe 100 is then re-connected for another attempt at step 610.

If the alarm registered a color change at step 612 (for example, to red), the needle is sunk into the patient at step 622. Decision block 624 checks for a color change in the alarm as the needle is sunk into the patient. If a color change is detected, the epidural space is found at step 626. The user may then continue the procedure. If no color change is detected, the user checks if the needle is in too deep at decision block 620. If the needle is too deep, the electronic syringe is removed at step 616 and discarded at step 604. If the needle is not too deep, the user continues to sink the needle into the patient at step 622.

FIG. 6 illustrates one example of a method of using the electronic syringe to locate an epidural space. Modifications may be made. For example, the needle may include an air escape. It may then be attached to the electronic syringe prior to inserting into the patient. When the ligamentum flavum is found, the method may then proceed as shown in FIG. 6 from step 612. Other modifications may be made as well.

It is also noted that the electronic syringe 100 does involve injecting air into the patient. However, the amount of air injected to the patient is less than 0.1 mL, which is much less than prior techniques by as much as 30 time. In addition, the electronic syringe 100 is reusable within the same procedure, and may even be used repetitively if an epidural space is not located initially.

In addition, the electronic syringe 100 may be used with any suitable needle and/or stylet tip. The switch 108 may be implemented as any mechanism configured to move an actuator to compress the diaphragm 216. The switch 108 may be implemented as a push-button, or any other suitable mechanism.

The foregoing description of an implementation has been presented for purposes of illustration and description. It is not exhaustive and does not limit the claimed inventions to the precise form disclosed. Modifications and variations are possible in light of the above description or may be acquired from practicing the invention. Note also that the implementation may vary between systems. The claims and their equivalents define the scope of the invention.

## Claims

1. A device for locating an epidural space comprising:
a connector for forming a hermetically sealed connection with a needle;
an air vessel formed by an air tank, a diaphragm, and a space in the needle when the needle is attached to the connector;
an pressure sensor configured to detect the air pressure in the air vessel;
an actuator coupled to a switch and positioned adjacent to the diaphragm, the actuator operable to compress the diaphragm when the switch is actuated by a user causing a reduction in volume in the air vessel; and
a circuit coupled to the pressure sensor and to an alarm, the circuit operable to receive a signal indicative of the air pressure sensed by the pressure sensor, and to compare the signal to a threshold, to output a first state to the alarm when the pressure is above the threshold, and to output a second state to the alarm when the pressure falls below the threshold.

2. The device of claim 1 where the connector is a Luer-type connector.

3. The device of claim 1 where the connector is configured for connection with a Tuohy needle.

4. The device of claim 1 where the needle is used with a stylet tip.

5. The device of claim 1 where the alarm is a visual alarm indicating a pressure change relative to the threshold by actuating a color change in a light-emitting device.

6. The device of claim 1 where the alarm is an audible alarm.

7. The device of claim 1 where air vessel has a volume of approximately 1.13 mL before the diaphragm is compressed and a volume of approximately 1.06 mL when the diaphragm is compressed.

8. A method for locating an epidural space comprising:
inserting a needle in a patient until sensing the ligamentum flavum;
forming a closed air vessel with an air tank and the space in the needle;
reducing the volume in the air vessel;
measuring the air pressure in the air vessel;
comparing the air pressure measurement with a threshold while advancing the needle into the patient; and
stopping the needle when the air pressure falls below the threshold.

9. The method of claim 8 where the step of reducing the volume includes compressing a diaphragm on the air tank.

10. The method of claim 8 where the step of inserting the needle includes using a stylet tip.

11. The method of claim 8 further comprising generating a first alarm indicating a pressure measurement greater than the threshold and a second alarm indicating an air pressure measurement less than the threshold.

12. The method of claim 11 where the first alarm is a light having a first color and the second alarm is a light having a second color.

13. The method of claim 11 where the steps of generating the first and second alarms includes generating first and second audible alarms.

14. A method for locating an epidural space comprising:
connecting a needle to an air tank;
forming a closed air vessel by inserting the needle connected to the air tank in a patient until sensing the ligamentum flavum;
reducing the volume in the closed air vessel;
measuring the air pressure in the air vessel;
comparing the air pressure measurement with a threshold while advancing the needle further into the patient; and
stopping the needle when the air pressure falls below the threshold.

15. The method of claim 14 where the step of reducing the volume in the closed air vessel includes compressing a diaphragm on the air tank.

16. The method of claim 14 where the step of inserting the needle includes using a stylet tip.

17. The method of claim 14 further comprising generating a first alarm indicating a pressure measurement greater than the threshold and a second alarm indicating an air pressure measurement less than the threshold.

18. The method of claim 17 where the first alarm is a light having a first color and the second alarm is a light having a second color.

19. The method of claim 17 where the steps of generating the first and second alarms includes generating first and second audible alarms.
